# EUROPEAN PATENT APPLICATION

(11) **EP 2 156 823 A1**
(43) Date of publication of application: **24.02.2010**
(21) Application number: 08162386.0
(22) Date of filing: 14.08.2008
(51) Int. Cl.: A61K 9/14, A61K 31/58

(54) **Process for the preparation of sterile powdered pharmeceutical compounds in the form of micro and nanoparticles**

(71) Applicant: Pharmatex Italia Srl, 20121 Milano (IT)
(72) Inventor: De Tommaso, Vincenzo, Dr., 20052, Monza (IT)
(74) Representative: Serravalle, Marco

(57) **Abstract**

The present invention relates to a process for the preparation of a sterile powdered pharmaceutical compound, the process comprising the following steps: a) the pharmaceutical compound is solubilized in a solvent and sterilized by filtration; b) the filtrate is collected in a sterile vessel in a class A room; c) the pharmaceutical compound is precipitated in a class A room; d) the precipitate suspended in a non-solvent is passed one or more times in a high pressure homogeniser.

The invention is also directed to triamcinolone acetonide according to this process, having a narrow particle size distribution and an average particle size by volume comprised between 0.001 µm and 50 µm.

## Description

The present invention concerns a process for the preparation of sterile powdered pharmaceutical compounds characterized by narrow particle size distribution. The process of the present invention makes use of sterilizing filtration through a 0.22 micron filter followed by treatment with a high pressure homogeniser.

Pharmaceutical compounds usually require to be stored in sterile conditions. Sterilization is performed through different methods, e.g. gamma rays or by use of ethylene oxide.

The use of gamma rays presents the important side effect of degrading part of the product. Furthermore it is an expensive and complex method. Ethylene oxide, on the other hand, requires high temperature and humidity. Also these conditions can cause degradation of the product.

W02007/051774 discloses a process for the preparation of sterile powdered pharmaceutical compounds, which process comprises: a) solubilization of the pharmaceutical compound in water or in an organic solvent at a concentration close to saturation; b) sterilizing filtration of the solution by passing it through a hydrophobic filter (0,22 microns membrane); c) evaporating the solvent at constant temperature while subjecting the solution to high intensity ultrasound.

There is the need for a process which not only results in a sterile powder, but which is also capable of producing a powdered compound with the desired particle size distribution.

The process of the present invention comprises the following steps:
a) the pharmaceutical compound is solubilized in a solvent and sterilized by filtration;
b) the filtrate is collected in a sterile vessel (class A room);
c) the pharmaceutical compound is precipitated;
d) the precipitate suspended in a non-solvent is passed one or more times in a high pressure homogeniser.

Preferably, in step c) the compound is precipitated by evaporation of the solvent and/or by addition of a non-solvent.

A preferred embodiment of this invention relates to a process for the preparation of a sterile powder pharmaceutical powder characterized by a narrow particle size distribution and by an average particle size by volume comprised between 0.001 µm and 50 µm, preferably between 0.005 µm and 25 µm. Preferably, the pharmaceutical powdered compound of the present invention is triamcinolone acetonide.

In another preferred embodiment of the invention, it is possible to influence the average particle size of the powdered compound by changing the process conditions in step d). The most important parameters which can be changed in step d are: operating pressure, number of passages and dimension of the interaction chamber.

The higher the operating pressure, the lower will be the average particle size of the powder. Preferably, an operating pressure of from 50 bar to about 3000 bar is used, more preferably form 200 to 2500 bar.

The number of passages can also influence the particle size of the powder. When leaving all other conditions unchanged, by increasing the number of passages it is possible to reduce the average particle size of the powder. In general, it is possible to use a number of passages comprised between 1 and 150, more preferably from 1 to 50.

The dimension of the interaction chamber is another parameter that can be varied in order to influence particle size distribution. A decrease in the size of the interaction chamber will lead to a decrease in the particle size of the powder. Interaction chambers having a size comprised from 30 µm to 300 µm are preferred, from 60 µm to 200 µm are most preferred.

As stated before, the process of the present invention produces, by changing the operative conditions of the homogenizer, powders having different average particle size.

In fact, by selecting proper process conditions for the high pressure homogenizer, it is possible to prepare a pharmaceutical powder, preferably triamcinolone acetonide, having conventional size, e.g. average size in volume comprised between 10 and 50 µm, as well as very fine powders with narrow particle size distribution, e.g. powders having average size in volume comprised between 0.05 and 1.00 µm, preferably from 0.10 µm to 0.70 µm and wherein 90 % by volume of the particles have a diameter equal to or lower than twice the average diameter by volume.

Step a) can be performed at room temperature, at high temperature or even at temperatures below room temperature. It is important that the room is classified as class A.

The filtration of step a) is preferably performed by using a nitrogen pressure of from 0.5 to 1.5 bar. The filtrate is collected in a sterile vessel in a class A room.

After precipitation, it is possible to pass the suspension directly into the homogenizer. Alternatively, the solvent is optionally removed, either partly or completely. In this case, it is important that the temperature is such that the product is stable and the solvent is volatile. In certain cases, it is possible to apply vacuum to maintain the temperature low.

After reduction of the particle size in the homogenizer, the powder is preferably dried for storage. The preferred drying method depends on the size of the particles. For particles larger than 1 µm, it is preferred to filter the suspension by using a 0.45 µm membrane. After filtration, the obtained solid is dried under vacuum.

In the case of nanoparticles, it is impossible to filter them on a membrane and the suspension is either directly dried under vacuum, or it concentrated by using ultrafiltration and then dried under vacuum.

The dried powder is preferably passed through a sieve with from 70 to 250 micron meshes. In this way, possible agglomerates are removed.

### Brief description of the drawings

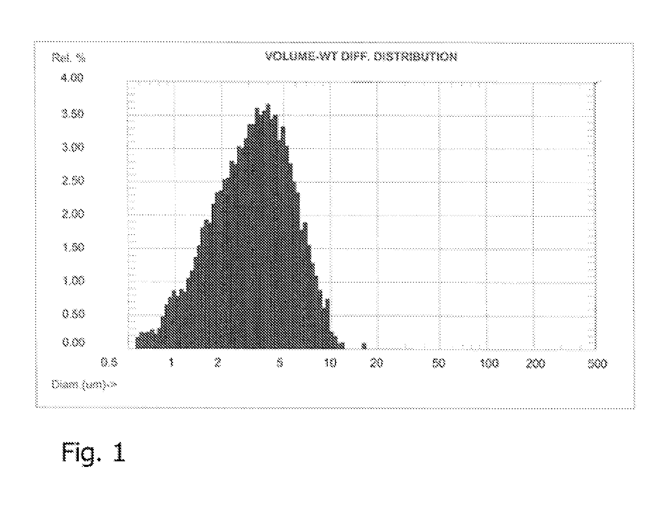

Fig. 1 shows the particle size distribution of the sterile powder of example 1.

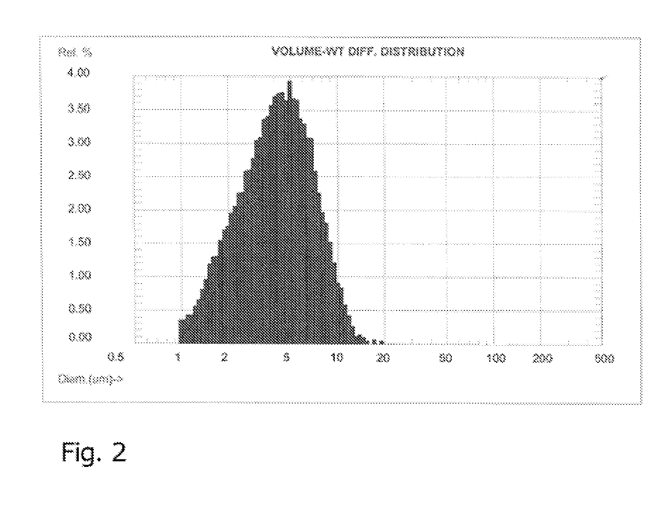

Fig. 2 shows the particle size distribution of the sterile powder of example 2.

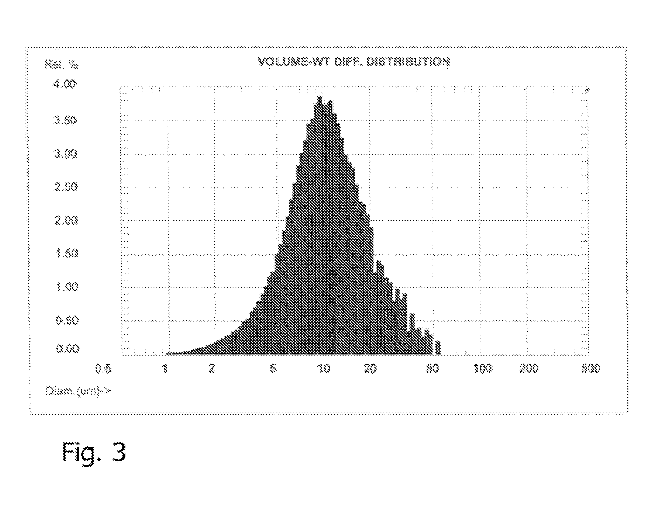

Fig. 3 shows the particle size distribution of the sterile powder of example 3.

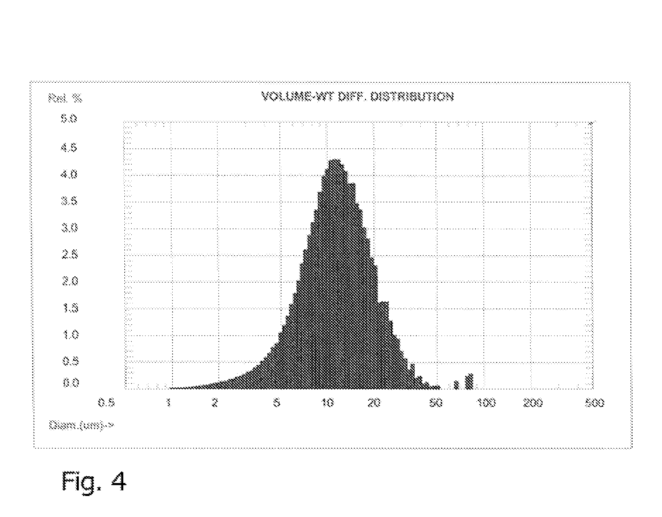

Fig. 4 shows the particle size distribution of the sterile powder of example 4.

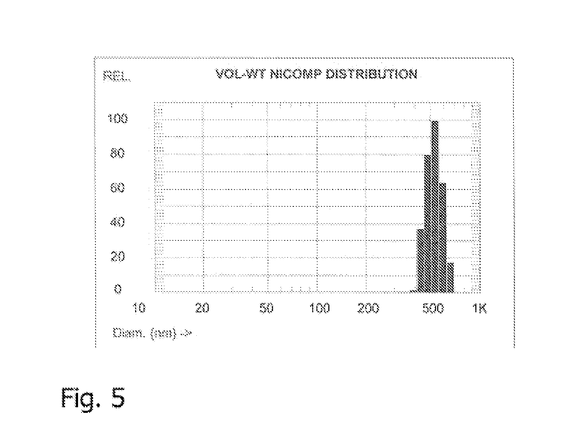

Fig. 5 shows the particle size distribution of the sterile powder of example 5.

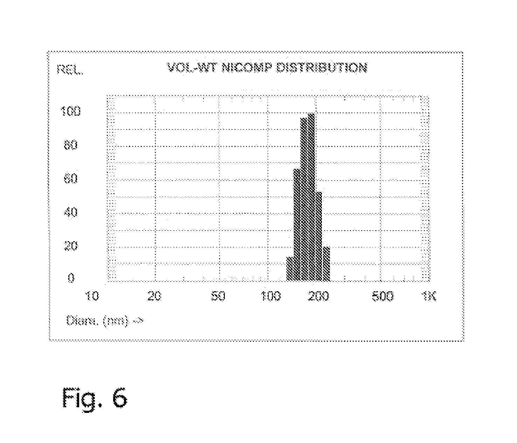

Fig. 6 shows the particle size distribution of the sterile powder of example 6.

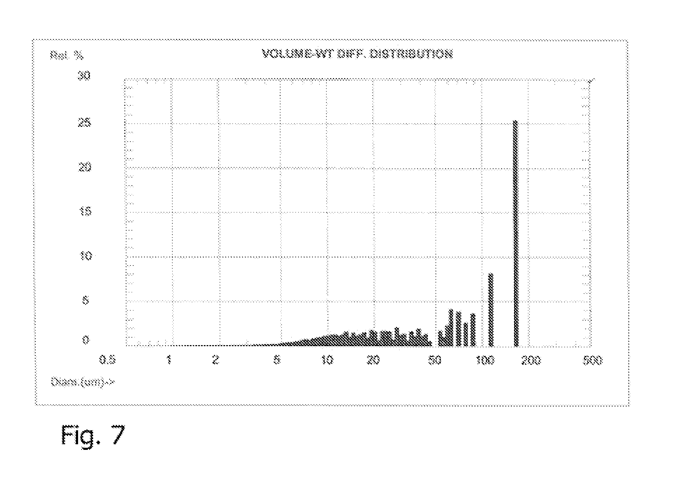

Fig. 7 shows the particle size distribution of the sterile powder of example 1 before passing the powder in the homogenizer.

### Examples

The particle size of the powdered compounds was measured by using a NICOMP 780 Accusizer in the case of particles larger than 1 µm. For particles having size from 1 nm to 1 µm, a NICOMP Submicron was used.

Triamcinolone acetonide used in the examples is commercially available from Farmabios.

### Example 1

15 g of triamcinolone acetonide were solubilized in 400 ml of acetone at 50-60 °C. The solution was filtered through a 0.22 µm sterilizing filter at 50 °C and the filtrate collected in a vessel, previously sterilized, while operating in a sterile room (class A). 1000 ml of water for injectable preparations were added at 25°C, under vigorous stirring, to avoid the formation of crystals larger than 200 µm. Acetone is evaporated by heating the suspension at 35°C under vacuum. The suspension of triamcinolone in water is kept under stirring and passed once through a high pressure homogenizer equipped with an interaction chamber of 200 µm and at a pressure of 400 bar. Then, the suspension was passed three times in the homogenizer equipped with an interaction chamber of 100 µm and at a pressure of 1800 bar. The suspension is filtered on a 0.45 µm membrane. The solid remaining on the filter is dried in a oven under vacuum. The dried powder is then passed on a 70 µm sieve to remove possible agglomerates.

### Example 2

15 g of triamcinolone acetonide were solubilized in 400 ml of acetone at 50-60 °C. The solution was filtered through a 0.22 µm sterilizing filter at 50 °C and the filtrate collected in a vessel, previously sterilized, while operating in a sterile room (class A). 670 ml of water for injectable preparations were added at 25°C, under vigorous stirring, to avoid the formation of crystals larger than 200 µm. The suspension of triamcinolone in acetone/water is kept under stirring and passed once through a high pressure homogenizer equipped with an interaction chamber of 200 µm and at a pressure of 400 bar. Then, the suspension was passed three times in the homogenizer equipped with an interaction chamber of 100 µm and at a pressure of 1800 bar. The suspension is filtered on a 0.45 µm membrane. The solid remaining on the filter is dried in a oven under vacuum. The dried powder is then passed on a 70 µm sieve to remove possible agglomerates.

### Example 3

15 g of triamcinolone acetonide were solubilized in 400 ml of acetone at 50-60 °C. The solution was filtered through a 0.22 µm sterilizing filter at 50 °C and the filtrate collected in a vessel, previously sterilized, while operating in a sterile room (class A). 1000 ml of water for injectable preparations were added at 25°C, under vigorous stirring, to avoid the formation of crystals larger than 200 µm. Acetone is evaporated by heating the suspension at 35°C under vacuum. The suspension of triamcinolone in water is kept under stirring and passed once through a high pressure homogenizer equipped with an interaction chamber of 200 µm and at a pressure of 400 bar. The suspension is filtered on a 0.45 µm membrane. The solid remaining on the filter is dried in a oven under vacuum. The dried powder is then passed on a 70 µm sieve to remove possible agglomerates.

### Example 4

15 g of triamcinolone acetonide were solubilized in 400 ml of acetone at 50-60 °C. The solution was filtered through a 0.22 µm sterilizing filter at 50 °C and the filtrate collected in a vessel, previously sterilized, while operating in a sterile room (class A). 1000 ml of water for injectable preparations were added at 25°C, under vigorous stirring, to avoid the formation of crystals larger than 200 µm. The suspension of triamcinolone in water/acetone is kept under stirring and passed once through a high pressure homogenizer equipped with an interaction chamber of 200 µm and at a pressure of 400 bar. The suspension is filtered on a 0.45 µm membrane. The solid remaining on the filter is dried in a oven under vacuum. The dried powder is then passed on a 70 µm sieve to remove possible agglomerates.

### Example 5

15 g of triamcinolone acetonide were solubilized in 400 ml of acetone at 50-60 °C. The solution was filtered through a 0.22 µm sterilizing filter at 50 °C and the filtrate collected in a vessel, previously sterilized, while operating in a sterile room (class A). 1000 ml of water for injectable preparations were added at 25°C, under vigorous stirring, to avoid the formation of crystals larger than 200 µm. Acetone is evaporated by heating the suspension at 35°C under vacuum. The suspension of triamcinolone in water is kept under stirring and passed once through a high pressure homogenizer equipped with an interaction chamber of 200 µm and at a pressure of 400 bar. Then, the suspension was passed three times in the homogenizer equipped with an interaction chamber of 100 µm and at a pressure of 1800 bar. Then, the homogenizer is equipped with an interaction chamber of 87 µm and the suspension is passed through the homogenizer 8 times at a pressure of 2000 bar. The suspension is then dried under vacuum. The obtained solid is passed on a 70 µm sieve.

### Example 6

15 g of triamcinolone acetonide were solubilized in 400 ml of acetone at 50-60 °C. The solution was filtered through a 0.22 µm sterilizing filter at 50 °C and the filtrate collected in a vessel, previously sterilized, while operating in a sterile room (class A). 1000 ml of water for injectable preparations were added at 25°C, under vigorous stirring, to avoid the formation of crystals larger than 200 µm. Acetone is evaporated by heating the suspension at 35°C under vacuum. The suspension of triamcinolone in water is kept under stirring and passed once through a high pressure homogenizer equipped with an interaction chamber of 200 µm and at a pressure of 400 bar. Then, the suspension was passed three times in the homogenizer equipped with an interaction chamber of 100 µm and at a pressure of 1800 bar. Then, the homogenizer is equipped with an interaction chamber of 87 µm and the suspension is passed through the homogenizer 22 times at a pressure of 2000 bar. The suspension is then dried under vacuum. The obtained solid is passed on a 70 µm sieve.

### Comparative example 1

Example 1 of WO 2007/051774 was repeated. In a 250 ml one neck round bottomed flask with a thermostatic jacket and a magnetic stirrer containing 140 ml of acetone, 2.5 g of triamcinolone acetonide were added. The suspension was heated to 50-60 °C under stirring until the solid was fully solubilized. The solution was then passed through a 0.22 micron filter and collected. The solution was kept at 25-30 °C under vacuum and acetone was evaporated, while the solution was subjected to ultrasound. After complete drying of acetone, the solid was passed through a 70 micron sieve.

Table 1 shows a comparison of the particle size distribution obtained in example 3, 4 and comparative example 1. It is evident that the use of a high pressure homogenizer leads to a narrower particle size distribution than obtained by using sonication.

**Table 1**

| | Ex. 3 | Ex. 4 | Comp. Ex. 1 |
|---|---|---|---|
| 10% | <4.69 µm | <5.52 µm | - |
| 20% | <6.15 µm | <7.23 µm | <7 µm |
| 50% | <10.03 µm | <11.13 µm | <18 µm |
| 60% | <11.75 µm | <12.96 µm | <19 µm |
| 90% | <22.46 µm | <21.47 µm | <35 µm |

## Claims

1. Process for the preparation of a sterile powdered pharmaceutical compound, the process comprising the following steps:
a) The pharmaceutical compound is solubilized in a solvent and sterilized by filtration;
b) the filtrate is collected in a sterile vessel in a class A room;
c) the pharmaceutical compound is precipitated in a class A room;
d) the precipitate suspended in a non-solvent is passed one or more times in a high pressure homogeniser.

2. Process according to claim 1 wherein in step c) the compound is precipitated by evaporation of the solvent and/or by addition of a non-solvent.

3. Process according to claim 1 wherein in step c) the compound is precipitated by addition of a non-solvent.

4. Process according to claims 1-3 wherein the non-solvent comprises water.

5. Process according to claims 1-3 wherein the homogenizer is operated at a pressure comprised between 50 and 3000 bar, preferably between 200 and 25000 bar.

6. Process according to claims 1-4 wherein the homogenizer is operated with an interaction chamber of from 30 µm to 300 µm, preferably from 60 µm to 200 µm.

7. Process according to claims 1-6 wherein the suspension is passed in the homogenizer from 1 to 150 times, preferably from 1 to 50 times..

8. Process according to claims 1-7 wherein the pharmaceutical compound is triamcinolone acetonide.

9. Triamcinolone acetonide obtainable according to the process of claim 8.

10. Triamcinolone acetonide according to claim 9, having a narrow particle size distribution and an average particle size by volume comprised between 0.001 µm and 50 µm, preferably between 0.005 µm and 25 µm.

11. Triamcinolone acetonide according to claim 10, having average size in volume comprised between 0.05 and 1.00 µm, preferably from 0.10 urn to 0.70 µm.

12. Triamcinolone acetonide according to claim 11, wherein 90 % by volume of the particles have a diameter equal to or lower than twice the average diameter by volume.
